# EUROPEAN PATENT APPLICATION

(11) **EP 2 444 118 A2**
(43) Date of publication of application: **25.04.2012**
(21) Application number: 11180507.3
(22) Date of filing: 08.09.2011
(51) Int. Cl.: A61N 1/368, A61N 1/372

(54) **Non-programming activation device for switching modes of an implantable medical device and methods for same**

(30) Priority: 22.10.2010 US 910613
(71) Applicant: PACESETTER, INC., Sylmar, CA 91392-9221 (US)
(72) Inventor: Hoberman, Katie, Winnetka, California 91306 (US); Fain, Eric S., Menlo Park, California 94025 (US); Pillay, Nicola, Sunnyvale, California 94087 (US)
(74) Representative: Noble, Nicholas

(57) **Abstract**

When a medical procedure is performed on a patient in whom an implantable medical device is implanted, the medical procedure may have undesired effects on the medical device, such as triggering a response that initiates therapy by the device that is unnecessary and potentially dangerous to the patient. Systems and methods may facilitate performing of such medical procedures on such patients by automatically reprogramming the medical device, monitoring for one or more detectable characteristics associated with the medical procedure to be performed, and automatically restoring normal operation of the IMD after the medical procedure is completed.

## Description

### BACKGROUND OF THE INVENTION

Embodiments of the present invention generally pertain to implantable medical devices and more particularly to a non-programmable activation device that switches modes of operation of an implantable medical device and methods for using the activation device.

An implantable medical device (IMD) is implanted in a patient to monitor, among other things, electrical activity of a heart and to deliver appropriate electrical therapy, as required. Implantable medical devices include pacemakers, cardioverters, defibrillators, implantable cardioverter defibrillators (ICD), neurostimulation devices, pain suppression devices, implantable spinal stimulators, implantable trauma stimulators, cardiac resynchronization therapy devices, ventricular-assist devices, implantable heart monitors, neurological stimulators, implantable drug pumps, cochlear implants, ultrasonic or electrical osteogenesis stimulators, and the like. The electrical therapy produced by an IMD may include pain suppression, pacing pulses, cardioverting pulses and/or defibrillator pulses to reverse arrhythmias (e.g., tachycardias and bradycardias) or to stimulate the contraction of cardiac tissue (e.g., cardiac pacing) to return the heart to normal sinus rhythm or to promote bone growth to accelerate healing of bone fractures, or to inject controlled amounts of fluids subcutaneously, or epidurally and the like.

During different medical procedures, such as diagnostic imaging, a surgical procedure or a clinical examination, the IMD can be exposed to electric field, static magnetic fields, gradient magnetic fields, pulsed radio frequency (RF) fields or combined field effects. When the IMDs are exposed to certain external fields, such fields may interfere with normal operation of the IMD. For example, RF waves may induce energy on implanted leads or reduce the energy of pacing pulses resulting in loss of pacing capture. For example, an external magnetic field may generate magnetic forces on the IMD and on the leads and electrodes of the IMD. The magnetic forces may induce electric charges or potential on the leads and electrodes. The electric charges can cause over- or under-sensing of cardiac signals in the electrodes and leads. For example, the charges may cause the electrodes and leads to convey signals to the IMD that are not cardiac signals, but are treated by the IMD as cardiac signals. In another example, the charges may induce significant noise in sensed cardiac signals such that cardiac signals that are representative of an abnormal cardiac event go undetected by the IMD.

Further, when patients who are implanted with an IMD are subjected to external electromagnetic interference, undesirable electric current and voltage could be induced by such interference and could create undesirable physiological effects, such as the induction of an arrhythmia or heat induced pain. Examples of IMD malfunctions have been traced to medical procedures, such as radiofrequency catheter ablation, electrocautary, dental procedures, magnetic resonance imaging (MRI) techniques, as well as other medical procedures.

Magnetic resonance (MR) imaging systems generate relatively strong magnetic fields. For example, some known commercial MR imaging systems create magnetic fields on the order of 0.5 to 3.0 Tesla. MR Imaging systems may generate external magnetic fields of different strengths, such as 0.5 Tesla, 0.7 Tesla, 1.0 Tesla, 1.2 Tesla, 1.5 Tesla, 3 Tesla, etc. Some IMDs may operate safely while in certain modes, when exposed to lower strength magnetic fields. However, when IMDs are exposed to higher strength magnetic fields, the IMDs may not reliably operate in a physiologic safe manner in some modes. In order to safely operate in some external magnetic fields, the IMDs may switch modes to an "MR safe mode" or a "magnet mode." The MR safe mode and magnet mode do not correspond to a particular pacing mode, but instead represents particular settings for certain parameters (e.g., pulse amplitude, pulse width, configuration, pacing mode, etc.).

Methods have been proposed to reduce the effects of interference by MRI systems, or other medical procedures, on implantable medical devices. Some of these methods include reducing the effects of interference on the lead itself alone, or in combination with, changing the IMD settings. For example, certain conventional leads have increased insulation surrounding the lead body, or have wires or conductors within the lead with reduced diameter to limit the effects of the RF fields. However, adding insulation or reducing the size of wires or conductors may increase the cost of the lead and may decrease the effectiveness of the IMD.

Another approach is to have a doctor or medical technologists specifically program the IMD immediately prior to the medical procedure to inhibit the IMD from delivering therapy in response to false positive of an abnormal physiological behavior. To perform the programming of the IMD, a trained programmer person manually loads the IMD with MR safe settings parameters. After the medical procedure is completed, the doctor or medical user must reprogram the IMD to the pre-procedure or patient related settings. Most doctors and medical examination personnel are not trained to operate an external IMD programming device. Instead, the doctors at the MRI facility and medical personnel are trained to perform a diagnostic imaging scan, a medical procedure or clinical examination. A separate group of doctors or medical personnel specialize in IMD implant, IMD programming and IMD related activity. Therefore, a trained IMD programmer person must also be present when a patient with an IMD undergoes certain types of scans, exams or procedures un-related to the IMD. The required presence of a trained IMD programmer person to support the IMD throughout a non-IMD related medical procedure, scan or exam adds to the cost of scan or exam procedure, and creates workflow burden. Additionally, a risk of introducing errors due to human interaction increases as an IMD is repeatedly reprogrammed before and after each scan, procedure or exam.

A need remains for an improved method and device that can safely interact with an IMD before and after non-IMD related procedures.

### SUMMARY

In accordance with one embodiment, a method is provided for changing settings of an implantable medical device (IMD) prior to a medical procedure. The method includes configuring an IMD to perform tasks based on saved settings in an active parameter fields. The method also includes loading the active parameter fields with patient related settings, where the patient related settings have been configured to treat abnormal physiological behavior of a patient. The method further includes storing medical procedure related settings in long term memory of the IMD. The procedure related settings correspond to a medical procedure that may potentially be performed on the patient. Additionally, the method includes utilizing a portable external non-programmable activator (NPA) device to transmit a safe mode command to the IMD prior to the start of a medical procedure. In response to the safe mode command, the IMD automatically reconfigures to a procedure safe mode by loading the procedure related settings into the active parameter fields and operating the IMD based on the procedure related settings. The instructions to execute the method may be stored in an IMD control module.

In accordance with one embodiment, a method is provided wherein upon receiving the safe mode command, the IMD performs a device state check to determine whether a present device state satisfies predetermined device state criteria. The IMD will not automatically reconfigure to the procedure safe mode when the present device state does not satisfy the predetermined device state criteria. The device state check may, by way of example, determine whether a present lead impedance satisfies lead impedance criteria.

In accordance with one embodiment, a method is provided wherein upon receiving the safe mode command, the IMD performs a patient state check to determine whether a present patient state satisfies predetermined patient state criteria. The IMD will not automatically reconfigure to the procedure safe mode when the present patient state does not satisfy the predetermined patient state criteria. The patient state check will determine whether at least one heart rate, pacing threshold, and a need for pacing satisfies a corresponding patient state criteria.

In accordance with one embodiment, a method for automatically switching the IMD between procedure related settings and patient related settings is provided. The method includes maintaining the IMD in the procedure safe mode until the IMD receives a procedure complete command from the NPA device. Also, the method includes removing the NPA device from a transmitting sensitive region, proximate to the IMD, during the medical procedure. The method excludes the NPA device transmitting the procedure related, or the patient related settings to the IMD.

In accordance with one embodiment, a method for automatically switching the IMD between procedure related settings and patient related settings may include at least one of the medical procedures of image scanning, a surgical procedure and a clinical examination. Further, the medical procedure may represent at least one of an MR scan, CT scan, NM scan, PET scan, ultrasound scan, an X-Ray scan, a hemodynamic exam, a physiologic exam, an intracardiac exam and an ablation procedure.

In accordance with one embodiment, the method for automatically switching the IMD between procedure related settings and patient related settings may include, after completion of the medical procedure, utilizing a non-programmable activator (NPA) device to transmit a procedure complete command to the IMD. The method may also include the IMD loading the patient related settings into the active parameter fields upon receiving the procedure complete command from the NPA device.

In accordance with one embodiment, an implantable medical device (IMD) is provided. The IMD may be configured to couple to a lead located proximate to a heart of a patient. The IMD includes a control module configured to perform IMD related functions based on settings saved in active parameter fields for the control module. The IMD also includes active parameter fields being initially loaded with patient related settings, the patient related settings being configured to treat abnormal physiologic behavior of a patient. The IMD includes long term memory to store procedure related settings related to a medical procedure that may potentially be performed on the patient. The IMD further includes a transceiver.

In accordance with one embodiment, the NPA device is provided. The NPA device transmits a safe mode command to the IMD. In response to the safe mode command, the IMD automatically reconfigures to a procedure safe mode by loading the procedure related settings into the active parameter fields and operating the IMD based on the procedure related settings. The IMD remains in the procedure safe mode until the IMD receives a procedure complete command transmitted from the NPA device.

In an optional embodiment, after completion of the medical procedure, the NPA device is utilized to transmit a procedure complete command to the IMD. The IMD then loads the patient related settings into the active parameter fields upon receiving the procedure complete command. The embodiment also includes interrogating the IMD by the NPA device for a current state of the IMD and present IMD status information on the NPA device to the user. The embodiment includes storing a plurality of procedure related settings in the memory of the IMD associated with different medical procedures wherein the NPA device informs the IMD through the safe mode command to automatically reconfigure to a corresponding procedure safe mode by loading a select set of the procedure related settings into the active parameter fields.

### BRIEF DESCRIPTION OF THE DRAWINGS

The drawings illustrate generally, by way of example, but not by way of limitation, various embodiments discussed in the present document.

Figure 1 illustrates an NPA device and an implantable medical device (IMD) coupled to a heart in accordance with one embodiment.

Figure 2 illustrates a block diagram of internal components of the NPA device shown in Figure 1 to switch an IMD into a procedure safe mode in accordance with an embodiment.

Figure 3 illustrates a block diagram of exemplary internal components of the IMD shown in Figure 1 to switch an IMD into a procedure safe mode in accordance with an embodiment.

Figure 4(a) illustrates a flow chart for a process to switch an IMD into a procedure safe mode in accordance with an embodiment.

Figure 4(b) Illustrates a flow chart to switch an IMD out of a procedure safe mode in accordance with an embodiment.

Figure 5 illustrates a flow chart for a process to query IMD status using the NPA to switch an IMD into a procedure safe mode in accordance with an embodiment.

Figure 6 illustrates a functional block diagram of an external device that is operated to interface with the IMD to switch an IMD into a procedure safe mode in accordance with an embodiment.

Figure 7 illustrates an embodiment for NPA device that can be used with multiple different procedures in accordance with an embodiment.

Figure 8 illustrates a distributed processing system in accordance with one embodiment.

### DETAILED DESCRIPTION

In the following detailed description, reference is made to the accompanying drawings which form a part hereof, and in which are shown by way of illustration specific embodiments in which the present invention may be practiced. These embodiments, which are also referred to herein as "examples," are described in sufficient detail to enable those skilled in the art to practice the invention. It is to be understood that the embodiments may be combined or that other embodiments may be utilized, and that structural, logical, and electrical variations may be made without departing from the scope of the present invention. The following detailed description is, therefore, not to be taken in a limiting sense, and the scope of the present invention is defined by the appended claims and their equivalents. In the description that follows, like numerals or reference designators will be used to refer to like parts or elements throughout. In this document, the terms "a" or "an" are used, as is common in patent documents, to include one or more than one. In this document, the term "or" is used to refer to a nonexclusive or, unless otherwise indicated. In this document, the term "patient related settings" refers to the settings of an IMD, prior to a medical procedure.

In the following detailed description, reference to medical procedures shall be given the broadest reasonable meaning. The term medical procedure shall include any activity directed at or performed on an individual with the object of improving health, treating disease or injury, or making a diagnosis and the like. For example, the medical procedure may be one where the patient is exposed to an electrical field, such as Electroneuronography, EEG topography, Electrical impedance tomography or the like. Similarly, the medical procedure may be one where the patient is exposed to a magnetic field, such as Magnetoencephalography, Magnetic therapy, Magnetic resonance imaging and the like. Alternatively, the medical procedure may involve introducing the individual to electromagnetic radiation, such as Scintillography, pulsed electromagnetic fields (PEMF) and the like. The medical procedure may further involve procedures that do not fall within the realm of conventional medicine. The medical procedures may also encompass surgical procedures, clinical examinations, therapies and the like. As used herein, the term normal is intended to encompass non-medical procedure conditions and operating parameters that correspond to non-medical procedure conditions. As used herein, the term "command" and the term "signal" may be used interchangeably.

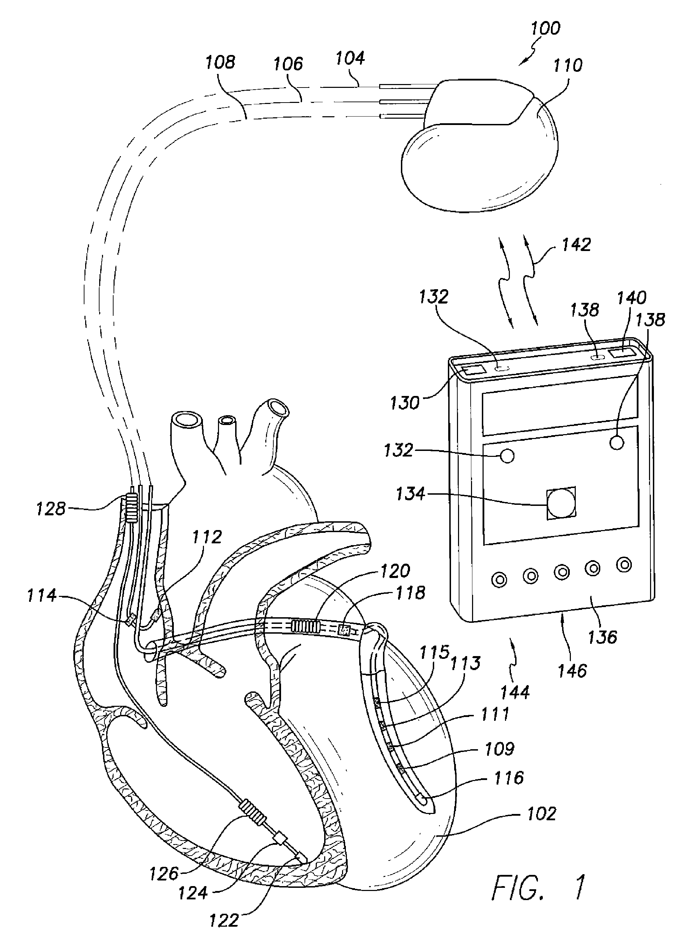

Figure 1 illustrates a non-programming activator (NPA) device 144 for use with an implantable medical device (IMD) 100 coupled to a heart 102, in accordance with an embodiment.

The IMD 100 may be a cardiac pacemaker, an ICD, a defibrillator, an ICD coupled with a pacemaker, a cardiac resynchronization therapy (CRT) pacemaker, a cardiac resynchronization therapy defibrillator (CRT-D), neurostimulators and the like. The IMD 100 includes a housing 110 that is joined to leads 104, 106, 108. The leads 104, 106, 108 are located at various locations of the heart 102, such as an atrium, a ventricle, or both, to measure cardiac signals of the heart 102. The leads 104, 106, 108 include the right ventricular (RV) lead 104, the right atrial (RA) lead 106, and the coronary sinus lead 108. Electrodes are provided on the leads 104, 106, 108 for sensing cardiac signals and/or for delivering stimulus or stimulation pulses to the heart 102. The housing 110 may be one of the electrodes and is often referred to as the "can", "case", or "case electrode."

The RV lead 104 includes an RV tip electrode 122, an RV ring electrode 124, and an RV coil electrode 126. The RV lead 104 may include a superior vena cava (SVC) coil electrode 128. The right atrial lead 106 includes an atrial tip electrode 112 and an atrial ring electrode 114. The coronary sinus lead 108 includes a left ventricular (LV) tip electrode 116, a left atrial (LA) ring electrode 118 and an LA coil electrode 120. Alternatively, the coronary sinus lead 108 may be a quadropole or mulitpole lead that includes multiple electrodes 109, 111, 113, 115 disposed within the left ventricle. Leads and electrodes other than those shown in Figure 1 may be included in the IMD 100 and positioned in or proximate to the heart 102.

The IMD 100 monitors cardiac signals of the heart 102 to determine if and when to deliver stimulus pulses to one or more chambers of the heart 102. The IMD 100 may deliver pacing stimulus pulses to pace the heart 102 and maintain a desired heart rate and/or shocking stimulus pulses to treat an abnormal heart rate such as tachycardia or bradycardia.

The IMD 100 includes a control module configured to perform IMD 100 related functions based on settings saved in active parameter fields for the control module. The active parameter fields are initially loaded with patient related settings configured to treat abnormal physiologic behavior of a patient. The IMD 100 also includes a long term memory to store procedure related settings that correspond to a medical procedure that may potentially be performed on the patient. The IMD 100 further includes a transceiver that transmits and receives signals from the NPA device 144.

The NPA device 144 is "non-programming" in that the device 144 does not have inputs, nor functionality, that allows a user to enter user-determined values for any IMD parameters. For example, unlike an IMD programmer, the NPA device 144 cannot be used to enter new values such as heart rate, pacing mode, sensing mode, stimulus strength, and the like. The NPA device 144 has limited functionality and is only able to change the IMD 100 between settings that are already stored in the IMD 100. The NPA device 144 cannot be used to enter new (un-stored) values for settings. The NPA device 144 has a limited user-interface, namely a user interface without a keyboard. The NPA device 144, in one embodiment, may be a portable handheld device that communicates with the IMD 100. The portable external NPA device 144 transmits a safe mode command to the IMD 100. The IMD 100, in response to the safe mode command, automatically reconfigures to a procedure safe mode by loading the procedure related settings into the active parameter fields. The IMD 100 operates based on the procedure related settings while the procedure is being performed on the individual. The IMD 100 remains in the procedure safe mode even when the NPA device 144 is removed from transmit sensitive region, proximate to the IMD 100.

In one embodiment, the IMD 100 remains in the procedure safe mode until the IMD 100 receives a procedure complete command transmitted from the NPA device 144. After completion of the medical procedure, the NPA device 144 is used to transmit a procedure complete command to the IMD 100. Upon receiving the procedure complete command from the NPA device 144, the IMD 100 loads the original patient related settings back into the active parameter fields from a memory.

Optionally, the IMD 100 may store a plurality of procedure related settings in the memory of the IMD 100 associated with different medical procedures. The NPA device 144 informs the IMD 100 through the safe mode command to automatically reconfigure to a corresponding procedure safe mode by loading a select set of the procedure related settings.

Optionally, the NPA device 144 may interrogate the IMD 100 for a current state of the IMD 100 and present IMD 100 status information on the NPA device 144 to the user.

The NPA device 144 includes a safe mode ON button 140 which when pressed sends a command 142 to the IMD 100 to switch active parameter fields to procedure related settings. The NPA device 144 also includes a safe mode OFF button 130 which when pressed sends a procedure complete command 142 to the IMD 100. In one embodiment the NPA device 144 includes a status request button 134 which when pressed sends a signal 142 to the IMD 100 requesting IMD 100 to relay the IMD's 100 status back to NPA device 144. The NPA device 144 further includes a label 136 to display information related to a medical procedure. For example, the label 136 may display the name of a medical procedure, for which the NPA device 144 is enabled. The NPA device 144 includes an indicator red light 132 and an indicator green light 138 to inform a user of NPA device 144 the status of communication sent and received from an IMD 100. Optionally, the NPA device 144 may include a speaker that allows users to communicate with NPA device 144 via audible sound. Optionally, the NPA device 144 includes an audio input device that takes audio command as input. The audio input activates the NPA device 144 to communicate a procedure related or status command from the NPA device 144 to the IMD 100. Optionally, the NPA device 144 may be formed as an integral part of another system or device or the like. For example, the NPA device 144 may be built into a MR scanner to send procedure related and status commands to the IMD 100 from the MRI system console.

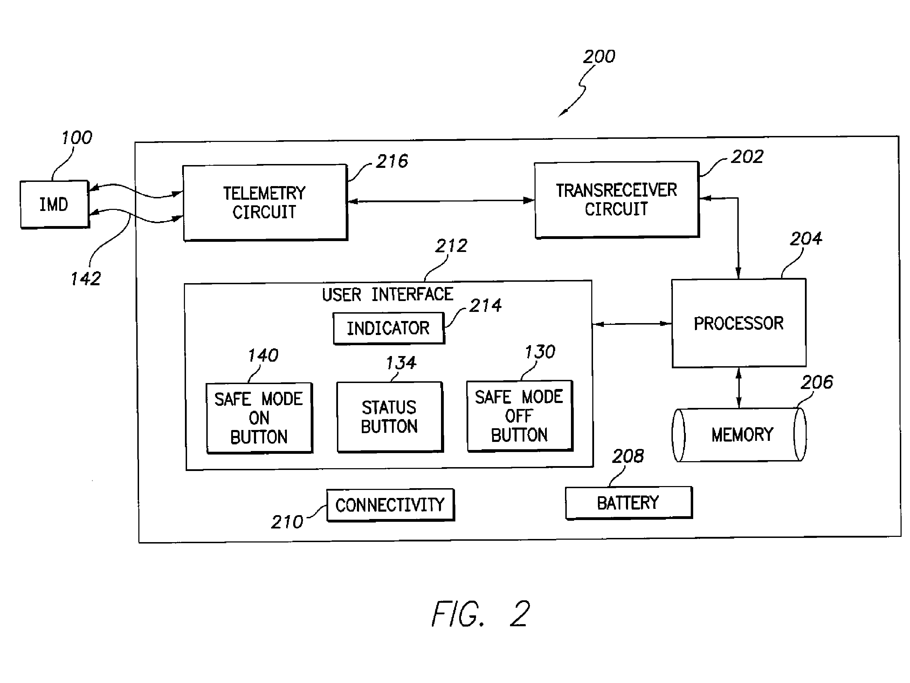

Figure 2 illustrates a block diagram 200 of typical internal components of the NPA device 144. The NPA device 144 includes a telemetry circuit 216, which enables the NPA device 144 to communicate 142 with the IMD 100. Although telemetry commonly refers to wireless communication mechanisms, the telemetry also encompasses data transferred over other media, such as a telephone or computer network, optical link or other wired communications. Many modern telemetry systems take advantage of the low cost and ubiquity of global system for mobile communications (GSM) networks by using short message service (SMS) to receive and transmit telemetry communication. For example, the NPA device 144 may communicate safe mode signal 142 or IMD 100 status request signal 142 to the IMD 100 via the telemetry circuit 216. Optionally, the NPA device 144 may also communicate with the programmer device via the telemetry circuit 216 to automatically program medical procedure related settings in the IMD 100.

The NPA device 144 further includes a transceiver circuit 202, which accepts signals from a transmission medium and decodes or translates the signals into a form that can drive local circuits. For example, the transceiver circuit 202 acts as a liaison between the telemetry circuit 216 and the processor 204.

The NPA device 144 includes a processor 204 to execute a sequence of stored instructions required to perform related programs. For example, the processor 204 accepts signal input from the transceiver circuit 202 or a user interface 212, executes related computer program and sends the output to components of the NPA device 144 that is in charge of executing the action. For example, an input command to switch safe mode parameters is issued by the user interface 212. The processor 204 identifies the command, processes the command and sends the code to the transceiver circuit 202 to send a signal to the IMD 100, via telemetry circuit, to switch active parameters with patient related settings or procedure related settings. In another example, the transceiver circuit receives the status signal from the IMD 100 and sends the signal to the processor 204. The processor 204 then processes the signal, executes the computer program that activates an appropriate indicator to communicate to the user the appropriate result. In one embodiment, the processor may be a low level embedded system. In another embodiment, the processor may be a complex system and may include microprocessors, control circuitry, memory, logic and timing circuit and other input or output circuits.

The NPA device 144 includes a memory 206 providing NPA device 144 the ability to store, retain, and recall information. For example, the NPA device 144 may store a temporary command in the memory 206 to be processed by the processor. Optionally, the memory 206 may store logs of communication between the NPA device 144 and the IMD 100. The memory 206 may be a short term storage memory in one embodiment and a long term memory in another embodiment.

The NPA device 144, in one embodiment, includes a user interface 212, allowing the user to interact with the NPA device 144. The user interface 212 provides a means of input, allowing the users to manipulate a system. For example, the user interface 212 includes a safe mode ON button 140, which sends a command to the IMD100 to switch active parameter fields to procedure related settings and store patient related settings in a section within a memory. The user interface 212 may also include a status button 134, which allows a user, of the NPA device 144, to get current state of the IMD 100. For example, the user interface 212 may include a safe mode OFF button 134, which commands the IMD 100 to switch the active parameter fields back to the patient related settings. The user interface also provides a means of output, allowing the system to indicate the effect of the user's manipulation or interaction with the system. For example, the user interface 212 of the NPA device 144 may include an indicator 214 that may communicate the status of the signal sent or received from the IMD 100 to user. The indicator, in one embodiment, may be green 138 and a red 132 light emitting diode (LED). Where the green 138 light may indicate that the communication between the NPA device 144 and the IMD 100 was successful. Alternatively, the red 132 light may indicate failed communication between the NPA device 144 and the IMD 100. For example, the user interface may include an indicator 214, a safe mode ON button 140, a safe mode OFF button 130, and a check status button 134. Alternatively, the user interface 212 may be a touch screen. In another embodiment, the user interface 212 may be audio controlled, e.g., the user interface may control the NPA device 144 by voice. In another embodiment, the user interface 212 may be a hybrid design including buttons, a touch screen, and/or audio control.

The NPA device 144 includes a battery 208, providing electric power for the operation of the NPA device 144. The battery 208 may be a disposable battery, designed to be used once and discarded when the batteries are exhausted, or the battery 208 may be a rechargeable battery, designed to be recharged and used multiple times.

The NPA device 144 includes a connectivity 210 module that provides multiple degrees of communication between the NPA device 144 and a clinical workflow. The clinical workflow may mean any form of communication between patient, clinicians, administrators, and families through the innovative use of the data, voice, and video. Alternatively, clinical workflow may be a process that accomplishes a definite step in an activity in the clinical care of patient.

The communication between the NPA device 144 and the clinical workflow may be via wired or wireless connectivity 210. The connectivity 210 between the NPA device 144 and the clinical workflow may be internet, a voice over IP (VoIP) gateway, a local plain old telephone service (POTS) such as a public switched telephone network (PSTN), a cellular phone based network, or the like. Alternatively, the connectivity 210 may be a local area network (LAN), a campus area network (CAN), a metropolitan area network (MAN), or a wide area network (WAN). The connectivity 210 module serves to provide a network that facilitates the transfer/receipt of information such as cardiac signal waveforms, fusion thresholds, fusion beat counts, total beat counts, IMD 100 programming data, safe mode signal to the IMD 100, and the like.

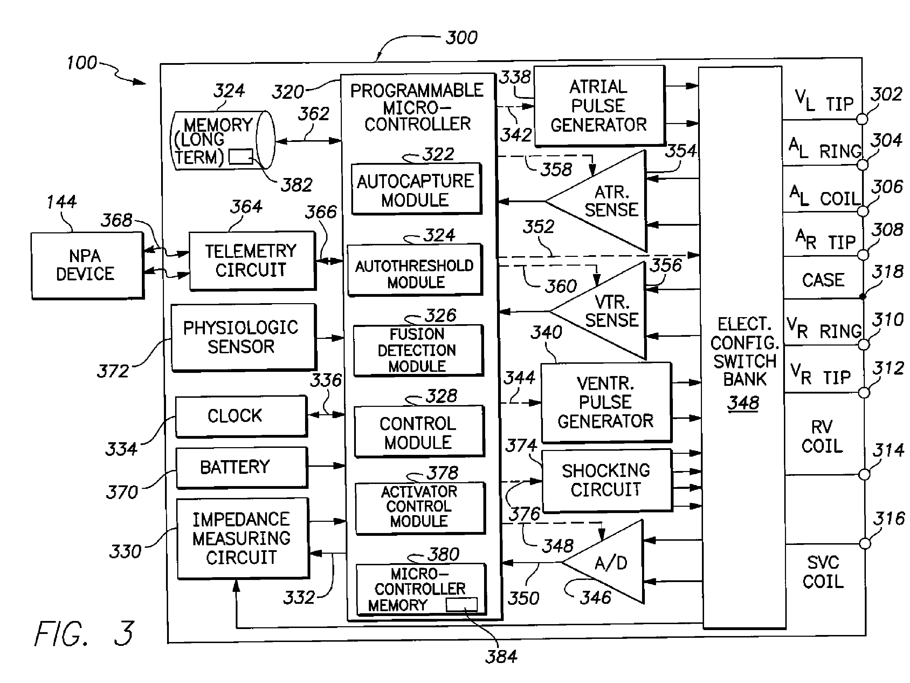

Figure 3 illustrates a block diagram of exemplary internal components of the IMD 100. The IMD 100 includes the housing 300 that includes a left ventricle tip input terminal (V_{L} TIP) 302, a left atrial ring input terminal (A_{L} RING) 304, a left atrial coil input terminal (A_{L} COIL) 306, a right atrial tip input terminal (A_{R} TIP) 308, a right ventricular ring input terminal (V_{R} RING) 310, a right ventricular tip input terminal (V_{R} TIP) 312, an RV coil input terminal 314 and an SVC coil input terminal 316. A case input terminal 318 may be coupled with the housing 300 of the IMD 100. The input terminals 302-318 may be electrically coupled with the electrodes 112-128 (shown in Figure 1).

The IMD 100 includes a programmable microcontroller 320, which controls the operation of the IMD 100. The microcontroller 320 (also referred to herein as a processor, processor module, or unit) typically includes a microprocessor, or equivalent control circuitry, and may be specifically designed for controlling the delivery of stimulation therapy and may further include RAM or ROM memory, logic and timing circuitry, state machine circuitry, and I/O circuitry. The microcontroller 320 may include one or more modules and processors configured to perform one or more of the operations described above in connection with the method 144, 250, and 300. An autocapture module 322 senses evoked responses of the heart 102 (shown in Figure 1) in response to delivery of stimulus pulses to the heart 102 when the IMD 100 operates in the autocapture mode described above. An autothreshold module 324 performs threshold searches when the IMD 100 operates in the autothreshold mode. For example, the autothreshold module 324 may incrementally decrease the electrical potential of stimulus pulses delivered to myocardium of the heart 102 (shown in Figure 1) until a loss of capture is detected in a first predetermined number of consecutive cardiac cycles. The autothreshold module 324 then may incrementally increase the electrical potential of the stimulus pulses until capture is detected in a second predetermined number of consecutive cardiac cycles.

A fusion detection module 326 identifies fusion-based behavior in myocardium of the heart 102 (shown in Figure 1). The fusion detection module 326 determines whether fusion occurs during a cardiac cycle. For example, the fusion detection module 326 may examine the position and/or shape of cardiac signal waveforms to identify fusion in a cardiac cycle, as described above. The fusion detection module 326 maintains the fusion beat count and the total beat count over predetermined intervals.

A control module 328 automatically switches the IMD 100 between the autothreshold and autocapture modes based on the presence of fusion-based behavior detected by the fusion detection module 326. For example, the control module 328 may switch the IMD 100 from the autocapture mode to the autothreshold mode when an amount of fusion-based behavior exceeds one or more fusion thresholds. In another example, the control module 328 switches the IMD 100 from the autothreshold mode to the autocapture mode when an amount of fusion-based behavior falls below one or more fusion thresholds. The control module 328 may base the decision whether to switch from one mode to the other on a number of cardiac cycles exhibiting fusion-based behavior, as described above.

An activator control module 378 is configured to control interfacing of the IMD 100 with the NPA device 144. The module 378 automatically switches between the IMD 100 patient related settings and procedure related settings based on a command received from the external NPA device 144. For example, the activator control module 378 may switch the IMD 100 from the patient related settings to the procedure related settings when the IMD 100 receives the safe mode command. The activator control module 378 may perform system check when the IMD 100 receives a status check command from the NPA device 144. For example, a user by pressing the status button on NPA device 144 sends a command to the IMD 100 to check lead impedance and also check the current settings of the IMD's 100 loaded in the active parameter fields. The activation control module processes the command, checks the system status, and transmits a signal back to the NPA device 144 via the telemetry circuit 364.

The microprocessor 320 receives signals from the electrodes 112-128 (shown in Figure 1) via an analog-to-digital (A/D) data acquisition system 346. The cardiac signals are sensed by the electrodes 112-128 and communicated to the data acquisition system 346. The cardiac signals are communicated through the input terminals 302-316 to an electronically configured switch bank, or switch, 348 before being received by the data acquisition system 346. The data acquisition system 346 converts the raw analog data of the signals obtained by the electrodes 120-138 into digital signals 350 and communicates the signals 350 to the microcontroller 320. A control signal 348 from the microcontroller 320 determines when the data acquisition system 346 acquires signals, stores the signals 350 in the memory 524, or transmits data and or signal to the NPA device 144.

The switch 348 includes a plurality of switches for connecting the desired electrodes 112-128 (shown in Figure 1) and input terminals 302-318 to the appropriate I/O circuits. The switch 348 closes and opens switches to provide electrically conductive paths between the circuitry of the IMD 100 and the input terminals 302-318 in response to a control signal 352. An atrial sensing circuit 354 and a ventricular sensing circuit 356 may be selectively coupled to the leads 104-108 (shown in Figure 1) of the IMD 100 through the switch 348 for detecting the presence of cardiac activity in the chambers of the heart 102 (shown in Figure 1). The sensing circuits 354, 356 may sense the cardiac signals that are analyzed by the microcontroller 320. Control signals 358, 360 from the microcontroller 320 direct output of the sensing circuits 354, 356 that are connected to the microcontroller 320. An impedance measuring circuit 330 is enabled by the microcontroller 320 via a control signal 332. The impedance measuring circuit 330 may be electrically coupled to the switch 348 so that an impedance vector between any desired pairs of electrodes 120-138 may be obtained. The IMD 100 additionally includes a battery 370 that provides operating power to the circuits shown within the housing 300, including the microcontroller 320. The IMD 100 includes a physiologic sensor 372 that may be used to adjust pacing stimulation rate according to the exercise state of the patient.

The clock 334 may measure time relative to the cardiac cycles or cardiac signal waveforms of the heart 102 (shown in Figure 1). The clock 334 measures elapsed amounts of time based on start and stop control signals 336 from the microcontroller 320 to determine the ventricular and atrial heart rates. Additionally, the clock 334 may track the amount of time elapsed between threshold searches. The elapsed time may be compared to a predetermined time period to determine whether to perform another threshold search, as described above.

The memory 324 may be embodied in a computer-readable storage medium such as a ROM, RAM, flash memory, or other type of memory. The microcontroller 320 is coupled to the memory 324 by a suitable data/address bus 362. The memory 324 may store programmable operating parameters and thresholds used by the microcontroller 320, as required. For example the stored programmable operating parameters and thresholds may be used to customize the operation of the IMD 100 to suit the needs of a particular patient. For example, the memory 324 may store the safe mode parameters used to switch the active parameters of the IMD 100 prior to a medical procedure. In another example, the memory 324 may store data indicative of cardiac signal waveforms, the fusion thresholds, predetermined time periods, fusion beat counts, total beat counts, and the like.

The safe mode parameters of the IMD 100 may be non-invasively programmed into the memory 324 through a telemetry circuit 364 in communication with the NPA device 144, such as a trans-telephonic transceiver or a diagnostic system analyzer. For example, the external device that telemetry circuit communicates with may be a NPA device 144. The telemetry circuit 364 is activated by the microcontroller 320 by a control signal 366. The telemetry circuit 364 allows intra-cardiac electrograms, cardiac waveforms of interest, detection thresholds, status information relating to the operation of IMD 100, and the like, to be sent to the NPA device 144 through an established communication link 368.

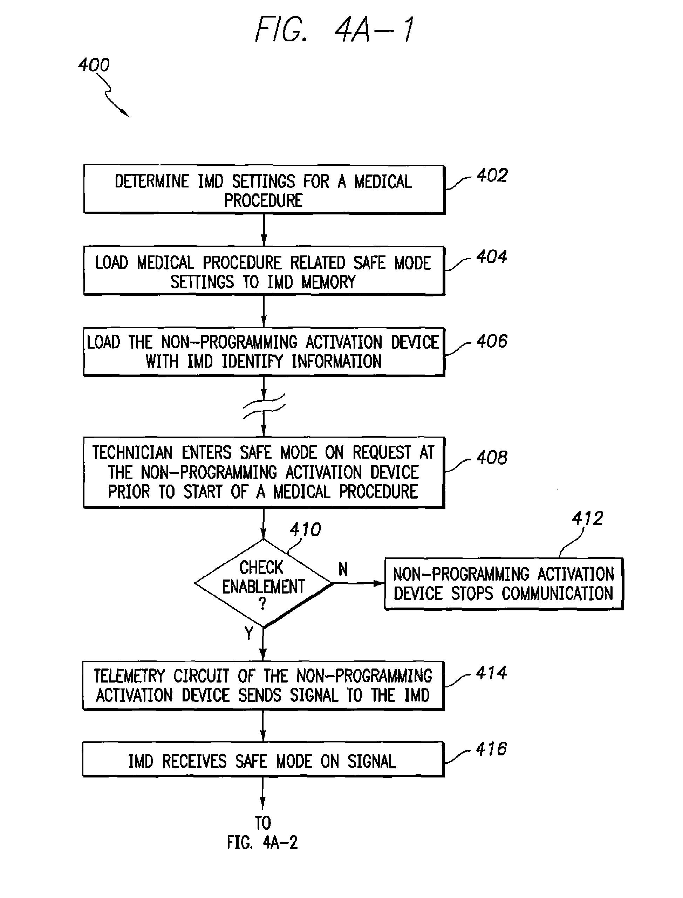

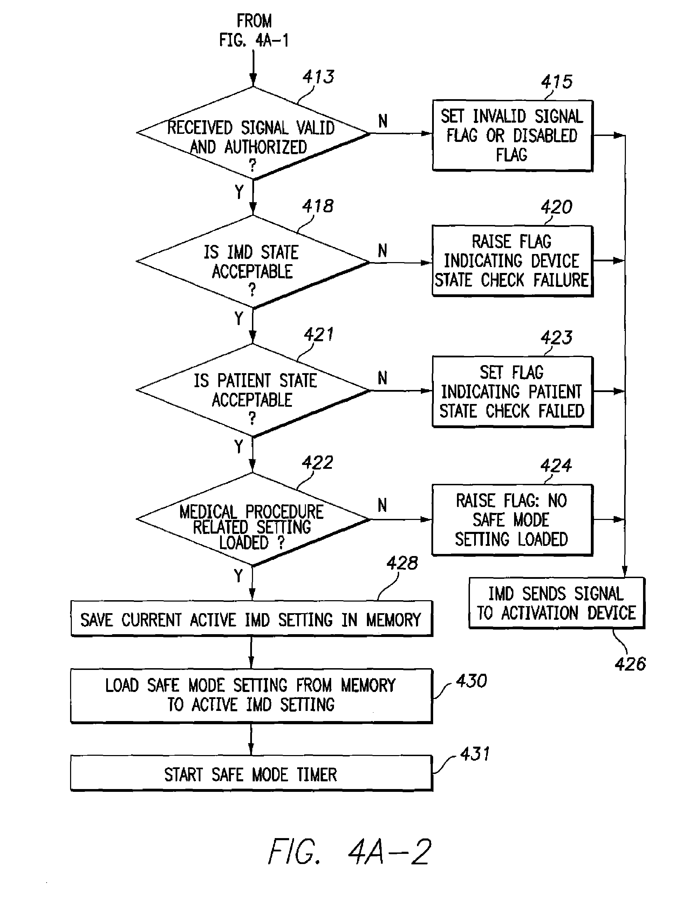

Figure 4(a) is a flow chart illustrating a process to switch an IMD into a procedure safe mode in accordance with an embodiment. The process 400 starts with a determination of which medical procedures or types of procedures the IMD 100 is to be configured 402. The determination of the procedure or procedure type includes determining one or more groups of settings for the parameters of the IMD 100. For example, the settings may include disabling sensing (e.g., DOO, pacing OFF), fixing the pacing rate at a predetermined rate (e.g., 80 bpm) and the like. The settings may include enabling atrial sensing and disabling ventricular sensing or vice versa, based on the medical procedure associated with the settings. At 404, the groups of settings associated with the medical procedure are loaded into a section 382 of a long-term memory 324 in the IMD 100 that is designated to store medical procedure related settings. Optionally, multiple groups of settings may be loaded in connection with multiple different medical procedures. For example, one group of settings may be loaded in connection with MRI procedures using up to 2 Tesla MRI scanners, while a second group of settings may be loaded in connection with MRI procedures using over 2 Tesla scanners. Third, fourth and/or fifth groups of settings may be loaded for surgical procedures, clinical examinations or emergency room situations, respectively. The medical procedure related settings are programmed for one or multiple medical procedures well in advance of the medical procedure. For example, the settings may be loaded at the time of the implant of an IMD 100 or by the manufacturer at at the time of manufacture of an IMD 100.

At 408, prior to the initiation of a medical procedure, a user utilizes the NPA device 144 to instruct the IMD 100 to switch the active parameter fields from patient related settings to a procedure safe mode. For example, the user may be a technician or a nurse practitioner. The user interface 212 provides a means of input, allowing the users to manipulate the NPA device 144. For example, the user interface 212 includes a safe mode ON button 140, which sends the IMD 100 command to switch the IMD's 100 active parameter fields from patient related settings to procedure related settings.

At 410, the NPA device 144 performs a system self check to verify if the NPA device 144 was enabled for the medical procedure. For example, the NPA device 144 may test if the NPA device 144 is wirelessly coupled to the IMD 100. Optionally, the NPA device 144 may refer to a medical procedure enablement checklist, to confirm if the NPA device 144 is enabled for the medical procedure. For example, the check list may be part of a database, accessible by the NPA device 144 via the connectivity 210 module. The NPA device 144 may look up the database to check if the medical procedure has been authorized by the clinic and the like. If the NPA device 144 is not enabled, the flow moves to 412. The process stops at 412 and the NPA device 144 ends communication. Optionally, the NPA device 144 may notify the user that the NPA device 144 is not enabled.

Returning to 410, if the NPA device 144 is enabled, the flow moves to 414. At 414, the NPA device 144 sends the command to switch the IMD 100 to procedure related settings. The NPA device 144 sends the signal via a telemetry circuit 414. Although telemetry commonly refers to wireless communication mechanisms, the telemetry also encompasses data transferred over other media, such as a telephone or computer network, optical link or other wired communications. Optionally, the telemetry system may use a GSM network by using SMS to receive and transmit telemetry communications. The IMD 100 receives the command to switch the active parameter fields to procedure related settings at 416 and the flow moves to 418.

In an alternate embodiment, the NPA device 144 may have a safety feature requiring the user to confirm if the IMD 100 should switch active parameter fields. For example, the NPA device 144 may request the user to press the safe mode ON button 140 a second time, as a confirmation step, to switch the active parameter fields to the procedure related settings.

At 413, the IMD 100 analyzes the received signal to determine whether the received signal is valid. For example, a header or data in the received signal may be analyzed to confirm that the received signal was transmitted from an authorized NPA device 144. The content of the received signal may be analyzed to confirm that the content corresponds to an instruction that the IMD 100 is capable of performing.

At 413, the IMD 100 also determines whether the IMD 100 is "enabled" to carry out NPA instructions in the received signal. For example, an external IMD programmer may be used separately to disable or enable NPA mode control of the IMD 100 by the NPA device 144. An IMD-trained physician or programmer may determine that it is not desirable for a particular patient's IMD 100 to be switchable to a procedure safe mode by an MR technician through the NPA device 144. Instead, the IMD-trained physician or programmer may want an IMD-trained person to be present when the IMD 100 is switched to the procedure safe mode. Hence, the IMD-trained physician or programmer disables an NPA enabled flag such as through an IMD programmer device. At 413, if the NPA enabled flag is set, the IMD 100 is enabled to switch modes in response to instructions from the NPA device 144 and flow moves to 418. If the NPA enabled flag is not set, the IMD 100 is not permitted to switch modes in response to instructions from the NPA device 144, and flow moves to 415.

At 415, an invalid signal flag and/or an NPA disabled flag is set. The invalid signal flag is set when the received signal is invalid as explained above. The NPA disabled flag is set when the IMD 100 determines at 413 that the NPA enabled flag is not set. Next, flow moves to 426, where the invalid signal flag or NPA disabled flag is transmitted back to the NPA device 144 to inform the user.

At 418, the IMD 100 performs one or more tests to check the state of the IMD 100 relative to predetermined device state criteria. For example, the device state check of the IMD 100 may perform a lead impedance test to verify if the lead impedance is within a predetermined range. Optionally, the device state test of the IMD 100 may test the sensing circuit or perform other diagnostics on the IMD 100. The lead impedance predetermined range may be set by the IMD's 100 manufacturer. Alternatively, the predetermined range may be prescribed by a clinician. Each lead is typically electrically isolated from other leads and is encased within an outer sheath that electrically insulates the lead conductors from body tissue and fluids. Cardiac leads are continuously flexed by the beating of the heart. Stress can also be applied to a lead body by patient movement, during implantation, during lead repositioning, or during IMD 100 changeout. Such stresses may lead to fracture of one or more conductors of the lead. Additionally, the electrical connection between the IMD 100 and the lead can be intermittently or continuously disrupted, which may result in intermittent or continuous changes in lead impedance.

The lead impedance test is one type of lead integrity test that may be performed by the IMD 100 at 418. Electrical lead integrity may be assessed soon after the IMD 100 receives the signal from the NPA device 144. If the lead impedance is uncharacteristically high, a lead fracture may be indicated. Detecting lead fractures is desirable because lead fractures may prevent delivery of effective therapeutic shocks to cardiac tissue. Alternatively, if the lead impedance is uncharacteristically low, this may indicate a breach in lead insulation.

Optionally, other types of device state checks may be performed on the IMD 100 and compared to corresponding device state criteria. If the test of the device state does not satisfy predetermined device state criteria, flow moves to 420 where the IMD 100 raises a fail flag. The fail flag indicates that the device state did not satisfy the predetermined device state criteria. When more than one device test is performed, multiple separate flags may exist. One or more flags may be set at 420 depending upon which device state checks are performed and which device state checks satisfy or do not satisfy the corresponding predetermined device state criteria. Optionally, values associated with the device state test(s) may be stored at 420 separately, or in combination, with the flags. Flow moves to 426 and the IMD 100 sends the fail flag(s) and/or values associated with the tests, to the NPA device 144. The process stops at 426 and the IMD 100 does not alter the active parameter fields if one or more device fail flags are raised.

Returning to 418, when the state of the IMD 100 is tested and determined to be within the predetermined device state criteria, flow moves to 421.

At 421, the IMD 100 performs one or more tests to assess a state of the patient relative to predetermined patient state criteria. For example, the assessment of the patient state may include measurements of one or more physiologic variables, such as heart rate, a need for pacing, sensing, the patient's pacing threshold and the like. The IMD 100 compares the measurements with predetermined patient state criteria. The predetermined patient state criteria may be set at the time of manufacture or programmed by a physician at the time of implant or thereafter. At 421, if it is determined that the patient state exceeds or falls below the one or more predetermined patient state criteria, then flow moves to 423.

At 423, the IMD 100 raises or sets one or more flags based on which patient state tests are performed and on whether the corresponding predetermined patient state criteria are satisfied. For example, patient heart rate may be too high, but the pacing threshold may be acceptable. In this example, the heart rate flag would be set/raised and the pacing threshold flag would not be set/raised. Optionally, the IMD 100 may store, at 423, the values measured at 421 (e.g., the heart rate, pacing threshold, etc.)

After 423, flow moves to 426 where the IMD 100 sends the flag(s) and/or measured value(s) to the NPA device 144.

Returning to 421, if it is determined that the patient state satisfies each of the predetermined patient state criteria then flow moves to 422. At 422, the IMD 100 performs another system check to verify if the prescribed medical procedure related settings are loaded in the section 382 of the memory 324. If the medical procedure related settings are not loaded, flow moves to 424 where the IMD 100 raises a non-procedures settings flag. At 426, the IMD 100 communicates the raised non-procedure settings flag to the NPA device 144. The process stops at 426 and the IMD 100 does not alter the active parameter fields if procedure related settings are not loaded.

Returning to 422, if the prescribed medical procedure settings are loaded in the memory 324, flow moves to 428. At 428, the IMD 100 stores the patient related settings from the active parameter fields to a section 384 of the micro-controller memory 380. Once patient related settings are saved in memory 380, the IMD 100 loads the procedure related settings into the active parameter fields. Thereafter, the IMD 100 operates based on the procedure related settings until the IMD 100 receives a command to switch the settings back to the original patient related settings.

Optionally, one or more of the checks performed at 418, 421 and 422 may be omitted entirely or the order changed.

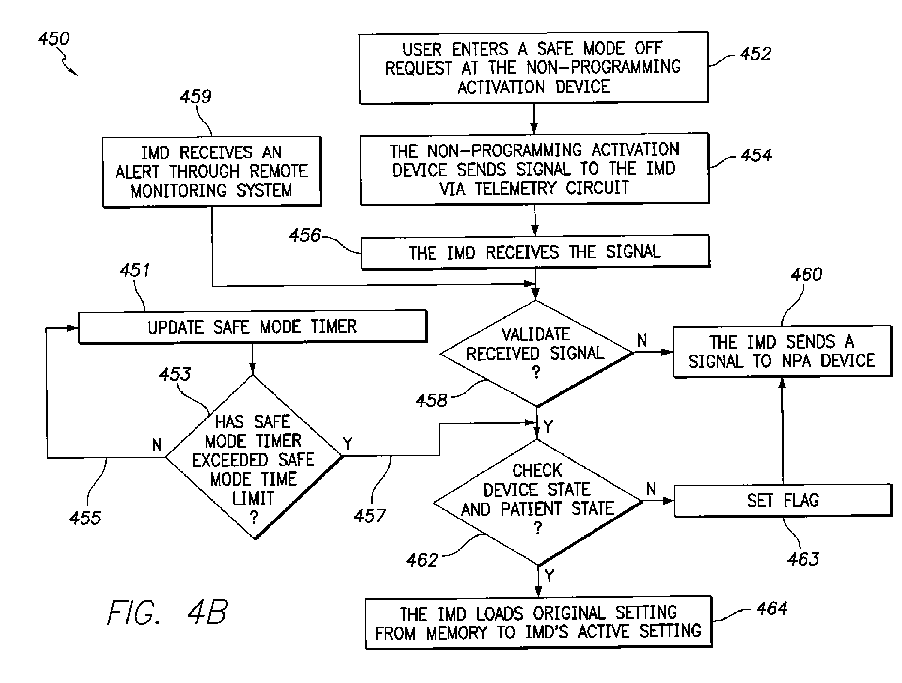

At 431, a safe mode timer is started. As explained below in connection with Figure 4(b), IMD 100 will automatically revert back to the patient related settings after a predetermined period of time has passed. For example, the IMD, 100 may have stored therein a safe mode time limit (e.g. 2 hours, 1 day, etc.). The IMD 100 will only remain in the procedure safe mode for the safe mode time limit which may be set at the time of manufacture or programmed at the time of implant or after implantation.

Figure 4 (b) Illustrates a flow chart to switch an IMD 100 out of a procedure safe mode in accordance with an embodiment. The process of Figure 4 (b) includes three parallel flow paths, namely 459 to 464, 452 to 464 and 451 to 464. The flow along 452 to 464 concerns the process to end the procedure safe mode at the command of the NPA device 144. The flow along 459 to 464 concerns the process to end the procedure safe mode in response to an alert from the remote monitoring system. The flow along 451 to 464 concerns the process to automatically end the procedure safe mode after a predetermined period of time.

At 451, the IMD 100 updates the safe mode timer after a set increment of time (e.g., 1 minute, 1 hour, etc.). At 453, the present value of the safe mode timer is compared to a safe mode time limit. If the current value of the safe mode timer does not exceed the safe mode time limit, the flow returns along 455 to 451, where the current value of the safe mode timer is incremented. At 453, when the safe mode timer exceeds the safe mode time limit, flow moves along 457 to 462 which is discussed below.

The flow along 452-462 concerns when a user utilizes the NPA device 144 to communicate a procedure complete command 452 to the IMD 100. For example, the user sends the procedure complete command 452 at the completion of a medical procedure. Alternatively, the user may send a procedure complete command prior to completion of a medical procedure.

At 454, the NPA device 144 transmits a command to the IMD 100, via the telemetry circuit 216, to switch out of safe mode. For example, the NPA device 144 may communicate procedure complete command 142 to the IMD 100 via the telemetry circuit 216.

At 456, the IMD 100 receives the procedure complete command from the NPA device 144. At 458, upon receiving the procedure complete command, the IMD 100 checks for the validity of the signal. The activator control module 378, which is configured to control interfacing of the IMD 100 with the NPA device 144, performs the check based on the instructions from the program microcontroller 320. For example, the user may mistakenly command to switch to procedure related settings when the IMD 100 is already in the safe mode. If the signal received at 456 is something other than the procedure complete command and the IMD 100 currently has procedure related settings loaded, the IMD 100 will send a communication back to the NPA device 144 indicating that the signal sent does not correspond to the correct next workflow step that the IMD 100 expects. When the received signal is not valid, the process stops at 460 and the IMD 100 does not alter the active parameter fields.

Returning to 458, if the signal received is a procedure complete command, the activator control module 378, commands the IMD 100 to perform a patient and device state check at 462. For example, the device and patient state checks may be similar to those discussed above in connection with Figure 4(a) at 418, 420, 421 and 423. For example, the device state check may assess the lead impedance, and the like, while the patient state check may assess the pacing threshold, heart rate and the like. If the device and/or patient state does not satisfy the assessment at 462, flow moves to 463 where a corresponding flag is set. Optionally, at 463, one or more values may be stored in connection with the checks performed at 462.

At 460, the flag(s) and/or measured value(s) are transmitted to the NPA device 144, along with a communication indicating that one or both of the device and patient state checks failed, and that the IMD 100 will remain in its current procedure safe mode. The process stops at 460 and the IMD 100 does not alter the active parameter fields.

Optionally, the check at 462 may be omitted entirely. Alternatively, the check at 462 may check only the device state or only the patient state, not both.

Returning to 462, if the device and patient state checks are passed, at 464 the activator control module 378 automatically switches the IMD 100 from the procedure related settings back to the patient related settings. For example, the IMD 100 loads the stored original patient related settings back into the active parameter fields from the microcontroller memory 380. Alternatively, the IMD 100 may load the stored original patient related settings into the active parameter fields from long term memory 362.

In an alternate embodiment, the IMD 100 may have a safety feature requiring the user to confirm if the IMD 100 should switch active parameter fields. For example, the IMD 100 may want the user to press the safe mode OFF button 138 a second time, as a confirmation step, to switch the IMD's 100 active parameter fields from procedure related setting back to the patient related settings stored in the IMD 100 memory 380.

At 459, the IMD 100 receives an alert communication, such as from a remote monitoring system (e.g., the Merlin.net patient care network or the Merlin@Home transmitter offered by S.J. Medical, Inc.). The alert command is verified at 458. When the alert command is verified, flow moves to 462, otherwise flow moves to 460 as discussed above.

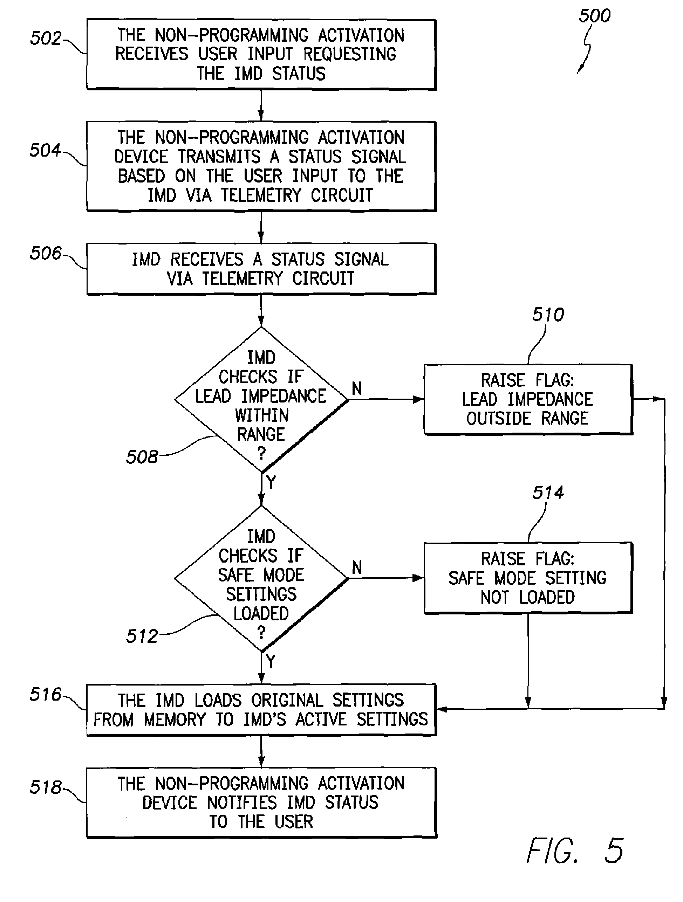

Figure 5 illustrates a flow chart for a process to query IMD 100 status, using the NPA device 144, to switch an IMD 100 into a procedure safe mode in accordance with an embodiment. Figure 5 describes the process for interrogating the IMD 100 using the NPA device 144 for the current state of the IMD 100, and presents the IMD 100 status information on the NPA device 144 to the user. The process 500 starts at 502 whereby a user requests the status of the IMD 100 using the NPA device 144. For example, the user may press a check status button 134 on the NPA device 144. Alternatively, the NPA device 144 may use audio signal as an input from the user, via the user interface 212. Alternatively, the user may input a request using a touch screen user interface 212. In another embodiment, the user may input a request using a hybrid of buttons, touch screen, and or audio control user interface 212.

The flow moves to 504 where the NPA device 144 communicates the system status request to the IMD 100 via the telemetry circuit 216. At 506 the IMD 100 receives a status check request from the NPA device 144. Upon receiving the status checks signal, at 508, the IMD 100 determines if the lead impedance is within a predetermined range. An impedance measuring circuit 330 is enabled by the activator control module 378 via a control signal 332. The impedance measuring circuit 330 may be electrically coupled to the switch 348 so that an impedance vector between any desired pairs of electrodes 120-138 may be obtained. The lead impedance range can be a predetermined range defined by the IMD's 100 manufacturer or a predetermined range prescribed by a clinician.

If the lead impedance is not within the predetermined range, flow moves to 510. At 510 the IMD 100 raises a lead impedance fail flag. Once the lead impedance fail flag is raised, flow moves to 516 and the IMD 100 sends the fail flag to the NPA device 144. The process stops at 518, where the NPA device 144 notifies user the status of the IMD 100. For example, the user interface 212 may include an indicator 214 that may communicate the status of the IMD 100 to user.

Returning to 508, when the IMD 100 lead impedance is within the predetermined range, flow moves to 512 and the IMD 100 performs another system check to verify if the prescribed medical procedure related settings are loaded in memory 324. If the medical procedure related settings are not loaded, flow moves to 514 where the IMD 100 raises a non-procedures settings flag. At 516, the IMD 100 communicates the raised non-procedure settings flag to the NPA device 144 via the telemetry circuit 364. The process stops at 518, where the NPA device 144 notifies user the status of the IMD 100. For example, the user interface 212 of the NPA device 144 may include an indicator 214 that may communicate the status of signal sent or received from the IMD 100 to the user. The indicator, in one embodiment, may be green 138 and a red 132 light emitting diode (LED). Where the green 138 light may indicate that the communication between the NPA device 144 and the IMD 100 was successful.

Returning to 512, if the prescribed medical procedure settings are loaded in the IMD 100 memory 324, flow moves to 516 where the IMD 100 sends the status signal to the NPA device 144 via the telemetry circuit 364. The status signal is used by the NPA device 144, to notify the user, that the IMD 100 passed system check test.

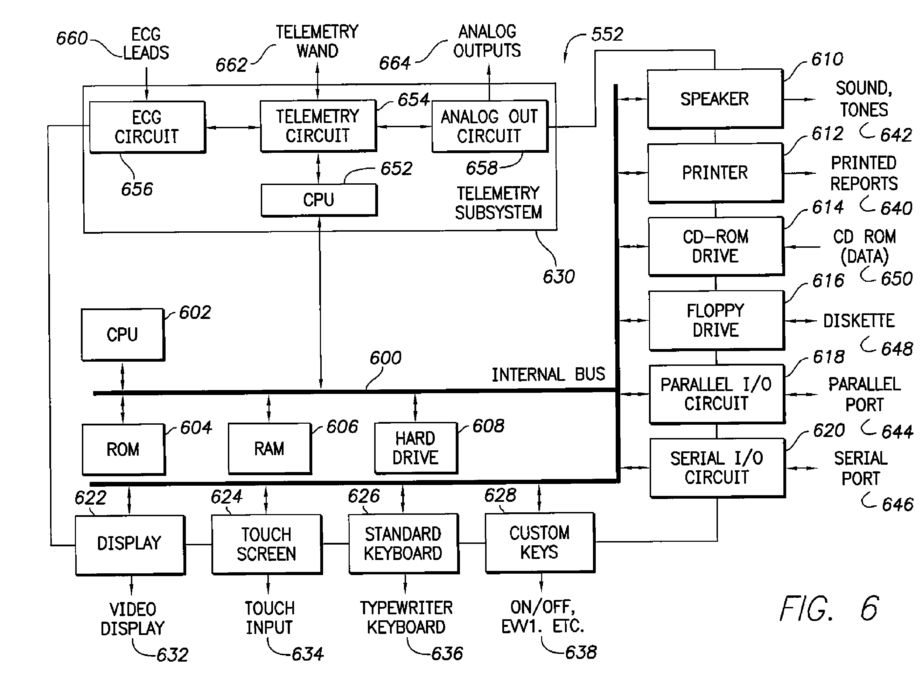

Figure 6 illustrates a functional block diagram of the external device 552, such as a programmer, that is operated to interface with the IMD 100 (shown in Figure 1). The external device 552 includes an internal bus 600 that connects/interfaces with a Central Processing Unit (CPU) 602, ROM 604, RAM 606, a hard drive 608, the speaker 610, a printer 612, a CD-ROM drive 614, a floppy drive 616, a parallel I/O circuit 618, a serial I/O circuit 620, the display 622, a touch screen 624, a standard keyboard connection 626, custom keys 628, and a telemetry subsystem 630. The internal bus 600 is an address/data bus that transfers information between the various components described herein. The hard drive 608 may store operational programs as well as data, such as waveform templates and detection thresholds.

The CPU 602 typically includes a microprocessor, a micro-controller, or equivalent control circuitry, is configured to control interfacing of the IMD 100 with the external device 552 and with the IMD 100 (shown in Figure 1). The CPU 602 may include RAM or ROM memory, logic and timing circuitry, state machine circuitry, and I/O circuitry to interface with the IMD 100. The display 622 (e.g., may be connected to the video display 632) with a touch screen 624 provides graphic information relating to the IMD 100. The touch screen 624 accepts a user's touch input 634 when selections are made. The keyboard 626 (e.g., a typewriter keyboard 636) allows the user to enter data to the displayed fields, as well as interface with the telemetry subsystem 630. Furthermore, custom keys 628 turn on/off 638 (e.g., EVVI) the external device 552. The printer 512 prints copies of reports 640 for a physician to review or to be placed in a patient file, and speaker 610 provides an audible warning (e.g., sounds and tones 642) to the user. The parallel I/O circuit 618 interfaces with a parallel port 644. The serial I/O circuit 620 interfaces with a serial port 646. The floppy drive 616 accepts diskettes 648. Optionally, the floppy drive 616 may include a USB port or other interface capable of communicating with a USB device such as a memory stick. The CD-ROM drive 614 accepts CD ROMs 650.

The telemetry subsystem 630 includes a central processing unit (CPU) 652 in electrical communication with a telemetry circuit 654, which communicates with both an ECG circuit 656 and an analog out circuit 658. The ECG circuit 656 is connected to ECG leads 660. The telemetry circuit 654 is connected to a telemetry wand 662. The analog out circuit 658 includes communication circuits to communicate with analog outputs 664. The external device 552 may wirelessly communicate with the IMD, 100 and utilize protocols, such as Bluetooth, GSM, infrared wireless LANs, HIPERLAN, 3G, satellite, as well as circuit and packet data protocols, and the like. Alternatively, a hard-wired connection may be used to connect the external device 552 to the IMD 100 (shown in Figure 1).

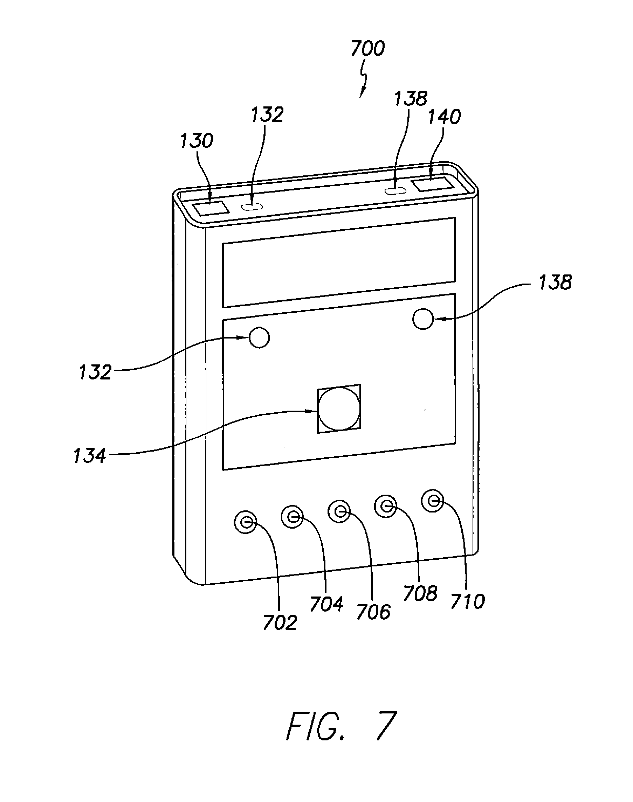

Figure 7 illustrates an embodiment for an NPA device 700 that can be used with multiple different medical procedures. The variation in types of diagnostic imaging or medical procedures that can affect the IMD 100 and the possibility of having multiple IMD's 100 in a patient is encompassed in Figure 7. Therefore, the IMD 100 may have one or more groups of settings loaded in the memory 324 of the IMD 100.

In one embodiment, the settings may include disabling sensing. For example, the NPA device 700 may command the IMD 100 to stop processing the electrical manifestation of naturally occurring heart beats as sensed by the heart electrodes. Furthermore, the NPA device 700 may command the IMD 100 to switch to a fixed pacing rate. The fixed pacing rate may be a predetermined rate (e.g., 80 bpm) and the like. The settings may include enabling atrial sensing and disabling ventricular sensing or vice versa, based on the medical procedure associated with the settings. For example, the settings may include parameters like single or dual chamber pacing, single chamber sensing or double chamber sensing or no sensing, or disabling rate response or single site pacing or multisite pacing (e.g., DOO, pacing OFF), and the like.

In one embodiment, the buttons 702,704,706,708, 710 on the NPA device 700 may be used to switch the IMD's 100 active parameter fields for different configuration of a diagnostic imaging. For example, the buttons 702,704,706,708, 710 may be used to switch the IMD's 100 settings for 0.7 Tesla, 1.0 Tesla, 1.2 Tesla, 1.5 Tesla, 3 Tesla MRI systems. Alternatively, the buttons 702,704,706,708, 710 on the NPA device 700 may be used to switch the IMD's 100 active parameter fields for a particular anatomy. For example, the buttons 702,704,706,708, 710 may be used to switch procedure related settings for pacemakers, cardioverters, defibrillators, implantable cardioverter defibrillators, implantable heart monitors respectively. Optionally, the NPA device 700 may be used to switch the settings of multiple IMD's manufactured by a vendor.

Optionally, the NPA device 700 may be personalized to a patient, whereby the NPA device 700 switches active parameter fields for the IMD's 100 in a particular patient. For example, if a patient has multiple IMD's 100 each button 702,704,706,708, 710 may be configured to switch the settings for a particular IMD 100. For example, the buttons 702,704,706,708, 710 may be used to switch procedure related settings for a pacemaker, pain suppression devices, implantable heart monitors, implantable drug pumps and neurological stimulators. Alternatively, the NPA device 700 may be personalized to a patient, whereby the NPA device 700 switches active parameter fields for different medical procedures that such patient may undergo.

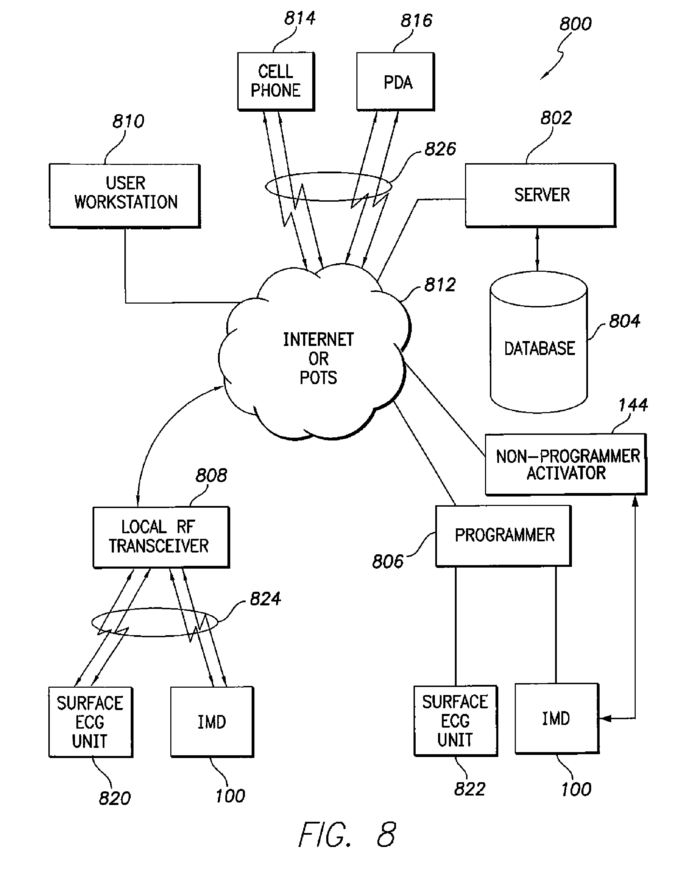

Figure 8 illustrates a distributed processing system 800 in accordance with one embodiment. The distributed processing system 800 includes a server 802 connected to a database 804, a programmer 806 (e.g., similar to external device 552 (shown in Figure 5)), a local RF transceiver 808, a NPA device 144 and a user workstation 810 electrically connected to a communication system 812. The communication system 812 may be the internet, a voice over IP (VoIP) gateway, a local plain old telephone service (POTS) such as a public switched telephone network (PSTN), a cellular phone based network, and the like. Alternatively, the communication system 812 may be a local area network (LAN), a campus area network (CAN), a metropolitan area network (MAN), or a wide area network (WAM). The communication system 812 serves to provide a network that facilitates the transfer/receipt of information such as cardiac signal waveforms, fusion thresholds, fusion beat counts, total beat counts, IMD 100 programming data, safe mode signal to the IMD 100, and the like.

The server 802 is a computer system that provides services to other computing systems over a computer network. The server 802 controls the communication of information such as cardiac signal waveforms, fusion thresholds, fusion beat counts, total beat counts, and the like. The server 802 interfaces with the communication system 812 to transfer information between the programmer 806, the local RF transceiver 808, the NPA device 144, the user workstation 810 as well as a cell phone 814 and a personal data assistant (PDA) 816 to the database 804 for storage/retrieval of records of information. On the other hand, the server 802 may upload a command to switch to procedure related settings from a NPA device 144 via the local RF transceiver 808 to an IMD 100.

The database 804 stores information such as cardiac signal waveforms, fusion thresholds, fusion beat counts, total beat counts, IMD 100 programming data, safe mode signal to the IMD 100, and the like, for a single or multiple patients. The information is downloaded into the database 804 via the server 802 or, alternatively, the information is uploaded to the server from the database 804. The programmer 806 is similar to the external device 552 and may reside in a patient's home, a hospital, or a physician's office. The programmer 806 interfaces with the surface ECG unit 822 and the IMD 100. The programmer 806 may wirelessly communicate with the IMD 100 and utilize protocols, such as Bluetooth, GSM, infrared wireless LANs, HIPERLAN, 3G, satellite, as well as circuit and packet data protocols, and the like. Alternatively, a hard-wired connection may be used to connect the programmer 806 to the IMD 100. The programmer 806 is able to acquire cardiac signals from the surface of a person (e.g., ECGs), intra-cardiac electrogram (e.g., IEGM) signals from the IMD 100, and/or cardiac signal waveforms, fusion thresholds, fusion beat counts, total beat counts, and the like, from the IMD 100. The programmer 806 interfaces with the communication system 812, either via the internet or via POTS, to upload the information acquired from the surface ECG unit 820 or the IMD 100 to the server 802.

The local RF transceiver 808 interfaces with the communication system 812 to upload one or more of cardiac signal waveforms, ventricular and atrial heart rates, and detection thresholds 246 (shown in Figure 2) to the server 802. In one embodiment, the surface ECG unit 820 and the IMD 100 have a bidirectional connection 824 with the local RF transceiver 808 via a wireless connection. The local RF transceiver 808 is able to acquire cardiac signals from the surface of a person, intra-cardiac electrogram signals from the IMD 100, and/or cardiac signal waveforms, fusion thresholds, fusion beat counts, total beat counts, and the like, from the IMD 100. On the other hand, the local RF transceiver 808 may download stored cardiac signal waveforms, fusion-thresholds, fusion beat counts, total beat counts, and the like, from the database 804 to the surface ECG unit 820 or the IMD 100.

The user workstation 810 may interface with the communication system 812 via the internet or POTS to download cardiac signal waveforms, fusion thresholds, fusion beat counts, total beat counts, NPA unit log and the like via the server 802 from the database 804. Alternatively, the user workstation 810 may download raw data from the IMD 100 via either the programmer 806 or the local RF transceiver 808. The user workstation 810 may download the information and notifications to the cell phone 814, the PDA 816, the local RF transceiver 808, the programmer 806, or to the server 802 to be stored on the database 804. For example, the user workstation 810 may communicate to the activation device 144 via a wireless communication link 826 to send the safe mode ON or procedure complete command to the IMD 100.

The NPA device 144 may communicate with the IMD 100 remotely via a local RF transceiver, local area network or wireless. Alternatively, the NPA device 144 may send IMD 100 status directly to a cell phone 814, PDA 816 or a workstation 810. Alternatively, the NPA device 144 may send IMD 100 status to a cell phone 814, PDA 816 or a workstation 810 via the server 802 from the database 804. Alternatively, the NPA device 144 may communicate with the programmer device to program procedure related settings if the NPA device 144 detected procedure related settings not loaded in IMD 100 memory.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the various embodiments of the invention without departing from their scope. While the dimensions and types of materials described herein are intended to define the parameters of the various embodiments of the invention, the embodiments are by no means limiting and are exemplary embodiments. Many other embodiments will be apparent to those of skill in the art upon reviewing the above description. The scope of the various embodiments of the invention should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc are used merely as labels, and are not intended to impose numerical requirements on their objects. Further, the limitations of the following claims are not written in means-plus-function format and are not intended to be interpreted based on 35 U.S.C. § 112, sixth paragraph, unless and until such claim limitations expressly use the phrase "means for" followed by a statement of function void of further structure.

The written description uses examples to disclose the various embodiments of the invention, including the best mode, and also to enable any person skilled in the art to practice the various embodiments of the invention, including making and using any devices or systems and performing any incorporated methods. The patentable scope of the various embodiments of the invention is defined by the claims, and may include other examples that occur to those skilled in the art. Such other examples are intended to be within the scope of the claims if the examples have structural elements that do not differ from the literal language of the claims, or if the examples include equivalent structural elements with insubstantial differences from the literal languages of the claims.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the various embodiments of the invention without departing from their scope. While the dimensions and types of materials described herein are intended to define the parameters of the various embodiments of the invention, the embodiments are by no means limiting and are exemplary embodiments. Many other embodiments will be apparent to those of skill in the art upon reviewing the above description. The scope of the various embodiments of the invention should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc are used merely as labels, and are not intended to impose numerical requirements on their objects. Further, the limitations of the following claims are not written in means-plus-function format and are not intended to be interpreted based on 35 U.S.C. § 112, sixth paragraph, unless and until such claim limitations expressly use the phrase "means for" followed by a statement of function void of further structure.

## Claims

1. A method for changing settings of an implantable medical device (IMD) prior to a medical procedure, the method comprising:
configuring an IMD to perform IMD related functions based on settings saved in active parameter fields for an IMD control module,
loading the active parameter fields with patient related settings, the patient related settings being configured to treat abnormal physiological behavior of a patient;
storing procedure related settings in memory of the IMD, the procedure related settings corresponding to a medical procedure that may potentially be performed on the patent;
prior to the medical procedure, utilizing a portable external non-programmable activator (NPA) device to transmit a safe mode command to the IMD; and
in response to the safe mode command, the IMD automatically reconfiguring to a procedure safe mode by loading the procedure related settings into the active parameter fields to enable operation of the IMD based on the procedure related settings.

2. The method of claim 1, further comprising at least one of:
a) maintaining the IMD in the procedure safe mode until one of a safe mode timer elapses, an instruction is received from a remote monitoring system, or the IMD receives a procedure complete command transmitted from the NPA device;
b) removing the NPA device from a transmit sensitive region proximate to the IMD during the medical procedure;
c) after completion of the medical procedure, utilizing the non-programmable NPA device to transmit a procedure complete command to the IMD, the IMD loading the patient related settings into the active parameter fields upon receiving the procedure complete command; and
d) interrogating the IMD by the NPA device for a current state of the IMD; and presenting IMD status information on the NPA device to the user.

3. The method of claim 1, further comprising: upon receiving the safe mode command, the IMD performing a device state check to determine whether a present device state satisfies predetermined device state criteria, wherein the IMD does not automatically reconfigure to the procedure safe mode when the present device state does not satisfy the predetermined device state criteria,
optionally wherein the device state check determines whether a present lead impedance satisfies lead impedance criteria.

4. The method of claim 1, further comprising: upon receiving the safe mode command, the IMD performing a patient state check to determine whether a present patient state satisfies predetermined patient state criteria, wherein the IMD does not automatically reconfigure to the procedure safe mode when the present patient state does not satisfy the predetermined patient state criteria,
optionally wherein the patient state check determines whether at least one heart rate, pacing threshold, and a need for pacing satisfies a corresponding patient state criteria.

5. The method of claim 1, wherein at least one of:
the NPA device does not transmit the procedure related settings, nor the patient related settings, to the IMD;
the medical procedure represents at least one of image scanning, a surgical procedure and a clinical examination; and
the medical procedure represents at least one of an MR scan, CT scan, NM scan, PET scan, ultrasound scan, an X-Ray scan, a hemodynamic exam, a physiologic exam, an intracardiac exam and an ablation procedure.

6. The method of claim 1, further comprising storing a plurality of procedure related settings in the memory of the IMD associated with different medical procedures; wherein the NPA device informs the IMD through the safe mode command to automatically reconfigure to a corresponding procedure safe mode by loading a select set of procedure related settings into the active parameter fields.

7. A system, comprising:
an implantable medical device (IMD) configured to be coupled to a lead located proximate to a heart of a patient, the IMD including:
a control module configured to perform IMD related functions based on settings saved in active parameter fields for the control module, the active parameter fields being initially loaded with patient related settings, the patient related settings being configured to treat abnormal physiologic behavior of a patient;
long term memory to store procedure related settings that correspond to a medical procedure that may potentially be performed on the patient; and
a transceiver; and
a portable external non-programmable activator (NPA) device, to transmit a safe mode command to the IMD; and
in response to the safe mode command, the IMD automatically reconfiguring to a procedure safe mode by loading the procedure related settings into the active parameter fields and operating the IMD based on the procedure related settings.

8. The system of claim 7, wherein the IMD remains in the procedure safe mode until one of a safe mode timer elapses, an instruction is received from a remote monitoring system or the IMD receives a procedure complete command transmitted from the NPA device.

9. The system of claim 7, wherein upon receiving the safe mode command, the IMD performing a device state check to determine whether a present device state satisfies predetermined device state criteria, wherein the IMD does not automatically reconfigure to the procedure safe mode when the present device state does not satisfy the predetermined device state criteria,
optionally wherein the device state check determines whether a present lead impedance satisfies lead impedance criteria.

10. The system of claim 7, wherein upon receiving the safe mode command, the IMD performing a patient state check to determine whether a present patient state satisfies predetermined patient state criteria, wherein the IMD does not automatically reconfigure to the procedure safe mode when the present patient state does not satisfy the predetermined patient state criteria,
optionally wherein the patient state check determines whether at least one heart rate, pacing threshold, and a need for pacing satisfies a corresponding criteria.

11. The system of claim7, wherein at least one of:
the IMD remains in the procedure safe mode even when the NPA device is removed from a transmit sensitive region, proximate to the IMD; and
the medical procedure represents at least one of the image scanning, a surgical procedure and a clinical examination.

12. The system of claim 7, wherein the medical procedure represents at least one of an MR scan, CT scan, NM scan, PET scan, ultrasound scan, an X-ray scan, a hemodynamic exam, a physiologic exam, an intracardiac exam and an ablation procedure.

13. The system of claim 7, wherein the IMD loads the patient related settings into the active parameter fields upon receiving a procedure complete command.

14. The system of claim 7, wherein the NPA device interrogates the IMD for a current state of the IMD; and present IMD status information on the NPA device to the user.

15. The system of claim 7, wherein the memory of the IMD stores a plurality of procedure related settings associated with different medical procedures; and wherein the NPA device informs the IMD through the safe mode command to automatically reconfigure to a corresponding procedure safe mode by loading a select set of the procedure related settings into the active parameter fields.
